# EUROPEAN PATENT SPECIFICATION

(11) **EP 0 528 858 B1**
(45) Date of publication and mention of the grant of the patent: **22.01.1997**
(21) Application number: 91908724.7
(22) Date of filing: 12.04.1991
(51) Int. Cl.: C07K 5/00, A61K 38/00

(54) **INHIBITORS OF DIPEPTIDYL-AMINOPEPTIDASE TYPE IV**
TYP-IV-DIPEPTIDYL-AMINOPEPTIDASE-INHIBITOREN
INHIBITEURS DE DIPEPTIDYL-AMINOPEPTIDASE DE TYPE IV

(30) Priority: 14.04.1990 US 510274
(43) Date of publication of application: 03.03.1993
(73) Proprietor: NEW ENGLAND MEDICAL CENTER HOSPITALS, INC., Boston, MA 02111 (US); TUFTS UNIVERSITY SCHOOL OF MEDICINE, Boston, MA 02111 (US)
(72) Inventor: BACHOVCHIN, William, W., Melrose, MA 02176 (US); PLAUT, Andrew, G. New England Medical Center, Boston, MA 02111 (US); FLENTKE, George R. Tufts University School of Med., Boston, MA 02111 (US)
(74) Representative: Deans, Michael John Percy
(86) International application number: US9102519
(87) International publication number: WO9116339

(56) References cited:
- WO-A-89/03223
- US-A- 4 935 493
- BIOCHEMISTRY, vol. 27, no. 20, 1988, Easton, PA (US); KETTNER et al., pp. 7682- 7688
- JOURNAL OF BIOLOGICAL CHEMISTRY, vol. 265, no. 7, March 1990, Baltimore, MD (US); BACHOVCHIN et al., pp. 3738-3743
- CHEMICAL ABSTRACTS, vol. 103, 1985, Columbus, OH (US); SHENVI et al., p. 689, no. 71709
- CHEMICAL ABSTRACTS, vol. 112, 1990, Columbus, OH (US); KETTNER et al., p. 80, no. 91790

## Description

This invention relates to the treatment of transplant rejection and of autoimmune diseases.

WO-A-8903223 discloses certain compounds of the general formula X-Pro-Y-boroPro, where X and Y are chosen from any amino acid (including proline). These compounds are inhibitors of the enzyme dipeptidyl amino peptidase type IV (DP IV).

DP IV is a serine protease present in many microbes, mammalian cells and tissues, for example, renal tubule cells, intestinal epithelium, and blood plasma. It is also present on the surface of CD-4+ and some CD-8+ T-cells, and in low amounts in the central nervous system. It is thought to be involved in the regulation of the immune response; occurrence of DP IV on a cell surface is associated with the ability of cells to produce interleukin 2 (IL-2). DP IV is also referred to as DAP IV or DPP IV; it is assigned EC number 3.4.14.5.

Three different inhibitors of DP IV are known. One of these is a suicide inhibitor: N-Ala-Pro-O-(nitrobenzyl-) hydroxylamine. (The standard three letter amino acid codes are used in this application; O represents oxygen.) Another is a competitive inhibitor: e-(4-nitro)benzoxycarbonyl-Lys-Pro. The third is a polyclonal rabbit anti-porcine kidney DP IV immunoglobulin.

The enzymatic activity of DP IV involves cleaving of a dipeptide from the free amino terminus of a polypeptide. DP IV has a preference for cleaving after a proline, i.e., a proline in the penultimate position from the amino terminus. A free amino terminus is required; thus, DP IV is a postproline cleaving enzyme with a specificity for removing an N-terminal W-Pro dipeptide from a polypeptide (where W can be any amino acid, including proline). DP IV also will remove a W'-Ala dipeptide from an amino terminus of a polypeptide when W' is an amino acid with a bulky side group, eg tyrosine.

We have sought to provide potent inhibitors of the enzymatic activity of DP IV. Generally, in certain of our compounds an α-amino boronic acid analog of proline (boroPro is used to designate one such analog which has the carboxyl group of proline replaced with a B(OH)₂ group, where (OH)₂ represents two hydroxyl groups and B represents boron) is bonded to an amino acid to form a dipeptide with boroPro as the C-terminal residue. We have found these dipeptide prolyl-boronic acids to be potent and highly specific inhibitors of DP IV activity and to have Ki values in the nanomolar range.

However, we have found that dipeptides having the boroPro moiety tend to be unstable; thus, we have designed inhibitors having at least two other amino acids. Generally, the structure of these inhibitors is X-Pro-Y-boroPro where X and Y are chosen from any amino acid (including proline). This tetrapeptide may be lengthened at its N-terminus by addition of one or more dipeptides, each dipeptide having the general formula Z-Pro or Z-ala, where each Z independently is any amino acid (including proline). This general structure is defined in more detail below. These inhibitors function as inhibitors of DP IV because each dipeptide portion is a substrate for DP IV and the final product of the reaction of an inhibitor with DP IV is the dipeptide inhibitor Y-boroPro. The amino terminus of these inhibitors must not be blocked or they lose their inhibitory capacity for DP IV, since DP IV cannot cleave a dipeptide from a blocked N-terminal polypeptide.

The invention features use of a compound having the formula 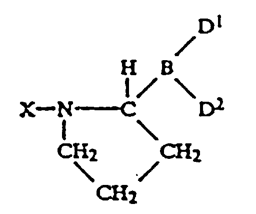 where each of D1 and D², independently, is a hydroxyl group or a group which is capable of being hydrolysed to a hydroxyl group in aqueous solution at physiological pH; and X comprises an amino acid or a peptide which mimics the site of a substrate recognised by a post prolyl cleaving enzyme for the preparation of a medicament for the treatment of transplant rejection or an autoimmune disease.

The invention is hereinafter more particularly described by way of example only with reference to the accompanying diagrams, in which:-
Figure 1 is a diagrammatic representation of the synthesis of a boro proline compound; and
Figure 2 is a diagrammatic representation of several embodiments of our compounds.

The structure of our inhibitory compounds is such that at least a portion of the amino acid sequence near the cleavage site of a DP IV substrate is duplicated, or nearly duplicated. This duplication is in part responsible for the ability of the inhibitory compounds to inhibit DP IV, by a mechanism thought to involve competitive inhibition between a DP IV inhibitory compound or DP IV cleavage product of the inhibitory compound, and the actual DP IV substrate.

The choice of amino acid sequence affects the inhibitory activity of the inhibitory compound, and its specificity. Peptide fragments can be synthesized and then tested to determine their efficacy as inhibitors, using standard techniques. Specificity is determined in a similar fashion, by testing the inhibitory effect of a particular inhibitory compound on the enzyme activity. The inhibitory compounds preferably inhibit the enzymatic activity of DP IV and do not inhibit enzymes necessary for normal cell functions.

The inhibitory compounds include a group 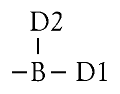 which causes the inhibitory compound to complex with DP IV, not only in a competitive fashion, but in a chemically reactive manner to form a strong bond between the inhibitory compound and DP IV. This group thus acts to bind the inhibitory compound to DP IV, and increases the inhibitory binding constant (Ki) of the inhibitory compound. Examples of such groups include boronates. These groups are covalently bonded to the prolyl residue of the compound, as in the above formula.

### Synthesis

### Synthesis of boroProline

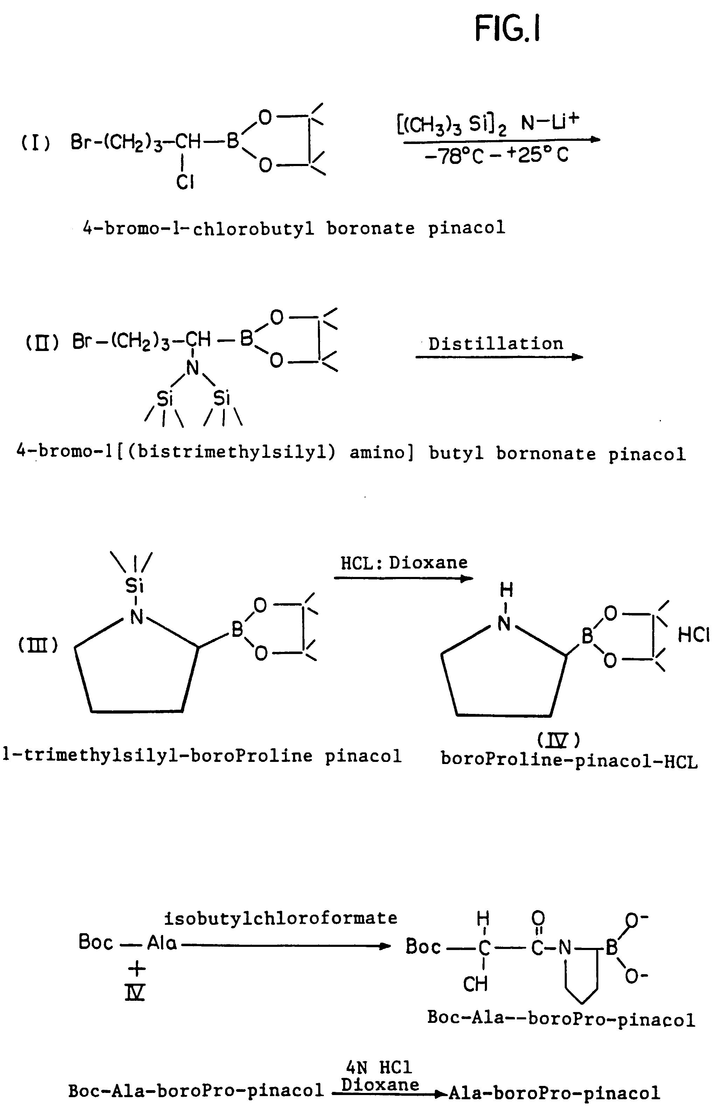

Referring to Figure 1, the starting compound I is prepared essentially by the procedure of Matteson et al., 3 Organometallics 1284, 1984, except that a pinacol ester is substituted for the pinanediol ester. Similar compounds such as boropipecolic acid and 2-azetodine boronic acid can be prepared by making the appropriate selection of starting material to yield the pentyl and propyl analogs of compound I. Further, Cl can be substituted for Br in the formula, and other diol protecting groups can be substituted for pinacol in the formula, e.g., 2,3-butanediol and alpha-pinanediol.

Compound II is prepared by reacting compound I with [(CH₃)O₃Si]₂N-Li+. In this reaction hexamethyldisilazane is dissolved in tetrahydrofuran and an equivalent of n-butyllithium added at -78°C. After warming to room temperature (20°C) and cooling to -78°C an equivalent of compound I is added in tetrahydrofuran. The mixture is allowed to slowly come to room temperature and to stir overnight. The alpha-bis[trimethylsilane]-protected amine is isolated by evaporating solvent and adding hexane under anhydrous conditions. Insoluble residue is removed by filtration under a nitrogen blanket, yielding a hexane solution of compound II.

Compound III, the N-trimethysilyl protected form of boroProline is obtained by the thermal cyclization of compound II during the distillation process in which compound II is heated to 100-150°C and distillate is collected which boils 66-62 Coat 8-13 Pa (0.06-0-10 mm) pressure.

Compound IV, boroProline-pinacol hydrogen chloride, is obtained by treatment of compound III with HCl:dioxane. Excess HCl and by-products are removed by trituration with ether. The final product is obtained in a high degree of purity by recrystallization from ethyl acetate.

The boroProline esters can also be obtained by treatment of the reaction mixture obtained in the preparation of compound II with anhydrous acid to yield 1-amino-4-bromobutyl boronate pinacol as a salt. Cyclization occurs after neutralizing the salt with base and heating the reaction.

### Example 1: Preparation of boroProline-pinacol (H-boroPro-pinacol)

The intermediate, 4-Bromo-1-chlorobutyl boronate pinacol, was prepared by the method in Matteson et al., Organometallics, (3): 1284-1288 (1984), except that conditions were modified for large scale preparations and the pinacol was substitued for the pinanedoil protecting group.

3-bromopropyl boronate pinacol was prepared by hydrogenboronation of allyl bromide (173 ml, 2.00 moles) with catechol borane (240 ml, 2.00 moles). Catechol borane was added to allyl bromide and the reaction heated for 4 hours at 100°C under a nitrogen atmosphere. The product, 3-bromopropyl boronate catechol (bp 95-102°C, 33 Pa (0.25 mm)), was isolated in a yield of 49% by distillation. The catechol ester (124 g, 0.52 moles) was transesterified with pinacol (61.5 g, 0.52 moles) by mixing the component in 50 ml of THF and allowing them to stir for 0.5 hours at 0°C and 0.5 hours at room temperature. Solvent was removed by evaporation and 250 ml of hexane added. Catechol was removed as a crystalline solid. Quantitative removal was achieved by successive dilution to 500 ml and to 1000 ml with hexane and removing crystals at each dilution. Hexane was evaporated and the product distilled to yield 177 g (bp 60 - 64°C, 0.35 mm).

4-Bromo-1-chlorobutyl boronate pinacol was prepared by homologation of the corresponding propyl boronate. Methylene chloride (50.54 ml, 0.713 moles) was dissolved in 500 ml of THF, 1.54 N n-butyllithium in hexane (480 ml, 0.780 moles) was slowly added at -100°C. 3-Bromopropyl boronate pinacol (178 g, 0.713 moles) was dissolved in 500 ml of THG, cooled to the freezing point of the solution, and added to the reaction mixture. Zinc chloride (54.4 g, 0.392 moles) was dissolved in 250 ml of THG, cooled to 0°C, and added to the reaction mixture in several portions. The reaction was allowed to slowly warm to room temperature and to stir overnight. Solvent was evaporated and the residue dissolved in hexane (1 liter) and washed with water (1 liter). Insoluble material was discarded. After drying over anhydrous magnesium sulfate and filtering, solvent was evaporated. The product was distilled to yield 147 g (bp 110 - 112°C, 27 Pa (0.200 mm)).

N-Trimethylsilyl-boroProline pinacol was prepared first by dissolving hexamethyldisilizane (20.0 g, 80.0 mmoles) in 30 ml of THF, cooling the solution to -78°C, and adding 1.62 N n-butyllithium in hexane (49.4 ml, 80.0 mmoles). The solution was allowed to slowly warm to room temperature. It was recooled to -78°C and 4-bromo-1-chlorobutyl boronate pinacol (23.9 g, 80.0 mmoles) added in 20 ml of THF. The mixture was allowed to slowly warm to room temperature and to stir overnight. Solvent was removed by evaporation and dry hexane (400 ml) added to yield a precipitant which was removed by filtration under an nitrogen atmosphere. The filtrate was evaporated and the residue distilled, yielding 19.4 g of the desired product (bp 60 - 62°C, 13-8 Pa (0.1 - 0.06 mm)).

H-boroProline-pinacol.HCl was prepared by cooling N-trimethylsilyl-boroProline-pinacol (16.0 g, 61.7 mmoles) to -78°C and adding 4 N HCL:dioxane 46 ml, 185 mmoles). The mixture was stirred 30 minutes at -78°C and 1 hour at room temperature. Solvent was evaporated and the residue triturated with ether to yield a solid. The crude product was dissolved in chloroform and insoluble material removed by filtration. The solution was evaporated and the product crystallized from ethyl acetate to yield 11.1 g of the desired product (mp 156.5 - 157°C).

### Synthesis of boroProline Peptides

General methods of coupling of N-protected peptides and amino acids with suitable side-chain protecting groups to H-boroProline-pinacol are applicable. When needed, side-chain protecting and N-terminal protecting groups can be removed by treatment with anhydrous HC1, HBr, trifluoroacetic acid, or by catalytic hydrogenation. These procedures are known to those skilled in the art of peptide synthesis.

The mixed anhydride procedure of Anderson et al., J. Am. Chem. Soc., 89:5012 (1984) is preferred for peptide coupling. Refering again to Figure 1, the mixed anhydride of an N-protected amino acid or a peptide varying in length from a dipeptide to tetrapeptide is prepared by dissolving the peptide in tetrahydrofuran and adding one equivalent of N-methylmorpholine. The solution is cooled to -20°C and an equivalent of isobutyl chloroformate is added. After 5 minutes, this mixture and one equivalent of triethylamine (or other sterically hindered base) are added to a solution of H-boroPro-pinacol dissolved in either cold chloroform or tetrahydrofuran.

The reaction mixture is routinely stirred for one hour at -20°C and 1 - 2 hours at room temperature (20°C). Solvent is removed by evaporation, and the residue is dissolved in ethyl acetate. The organic solution is washed with 0.20 N hydrochloric acid, 5% aqueous sodium bicarbonate, and saturated aqueous sodium chloride. The organic phase is dried over anhydrous sodium sulfate, filtered, and evaporated. Products are purified by either silica gel chromatography or gel permeation chromatography using Sephadex™ LH-20 and methanol as a solvent.

Previous studies have shown that the pinacol protecting group can be removed in situ by preincubation in phosphate buffer prior to running biological experiments; Kettner et al., J. Biol. Chem. 259: 15106-15114 (1984). Several other methods are also applicable for removing pinacol groups from peptides including boroProline and characterizing the final product. First, the peptide can be treated with diethanolamine to yield the corresponding diethanolamine boronic acid ester, which can be readily hydrolyzed by treatment with aqueous acid or a sulfonic acid substituted polystyrene resin as described in Kettner et al., id. Both pinacol and pinanediol protecting groups can be removed by treating with BC13 in methylene chloride as described by Kinder et al., J. Med. Chem., 28: 1917. Finally, the free boronic acid can be converted to the difluoroboron derivative (-BF2) by treatment with aqueous HF as described by Kinder et al., id.

Similarly, different ester groups can be introduced by reacting the free boronic acid with various di-hydroxy compounds (for example, those containing heteroatoms such as S or N) in an inert solvent.

### Example 2: H-Ala-boroPro

Boc-Ala-boroPro was prepared by mixed anhydride coupling of the N-Boc-protected alanine and H-boroPro prepared as described above. H-Ala-boroPro was prepared by removal of the Boc protecting group at 0°C in 3.5 molar excess of 4 N HCl-dioxane. The coupling and deblocking reactions were performed by standard chemical reaction. Ala-boroPro has a Ki for DP IV of -1 x 10⁻⁹M. Boc-blocked Ala-boroPro has no affinity for DP IV.

The two diastereomers of H-Ala-boroPro-pinacol can be partially separated by silica gel chromatography with 20% methanol in ethyl acetate as eluant. The early fraction appears by NMR analysis to be 95% enriched in one isomer. Because this fraction has more inhibitory power against DP IV than later fractions (at equal concentrations) it is probably enriched in the L-boroPro isomer.

One significant drawback with H-Ala-boroPro as an inhibitor for DP IV is that it decomposes in aqueous solution at neutral pH and room temperature (20 - 25°C) with a half-life of around 0.5 hour. Many dipeptide derivatives with a free N terminal amino group and a functional group (such as a difluoromethyl ketone) on the C-terminus are similarly unstable due to intramolecular reaction. A six member ring is formed between the amino and C-terminal functional groups and undergoes subsequent further reaction, such as hydrolysis. DP IV bound inhibitor is more stable, consistent with the hypothesis that decomposition is due to an intramolecular reaction.

H-Pro-boroPro is more stable than H-Ala-boroPro. The Ki of H-Pro-boroPro for DP IV is about 1 x 10⁻⁸M, and it decomposes in aqueous solution at room temperature (20-25°C) with a half life of about 1.5 hours. Although the affinity of H-Pro-boroPro is about 10-fold less than that of H-Ala-boroPro, the increased stability is advantageous.

Because of the relatively short half life of the above dipeptides inhibitory compounds of the invention are formed as tetrapeptides or longer peptides as shown in the general formula above. These inhibitory compounds are substrates for DP IV yielding the dipeptide inhibitor W-boroPro. These tetrapeptide boronic acids are generally stable and can be administered by any standard procedure to act as a substrate for DP IV and then as a source of a potent DP IV inhibitor. The advantages of such tetrapeptides is that inhibitor is released only in the vicinity of active DP IV. These tetrapeptide boronic acids can be made by the method of mixed anhydride coupling by one of ordinary skill in the art, e.g., Mattason, Organametallics 3:1284 to 1288, 1984.

### Test Systems

The following are examples of systems by which the inhibitory activity of the above described inhibitory compounds can be tested on DP IV. As an example H-Ala-boroPro is used to test each of these systems. Inhibitory compounds can be tested by simply substituting them for H-Ala-boroPro.

DP IV is purified from pig kidney cortex by the method of Barth et al., Acta Biol. Med. Germ. (1974) 32:157, and Wolf et al., Acta Biol. Med. Germ. (1978) 37:409, and from human placenta by the method of Puschel et al., E. Eur. J. Biochem. (1982) 126:359. H-Ala-boroPro inhibits both enzymes with a Ki of -1.0 x 10⁻⁹M.

### Human Peripheral Blood Mononuclear Cells

H-Ala-boroPro was tested for its influence on PHA-induced proliferation of human peripheral blood mononulcear cells. Human peripheral blood mono-nuclear cells were obtained from healthy human donors by Ficoll-Hypaque density gradient centrifugation. The cells are washed three times in RPMI 1640 medium and resuspended to a concentration of a 1 X 10⁶ in RPMI. 10% human serum was used as necessary.

The proliferative response of lymphocytes was measured using 3H-Thymidine incorporation. MNC cells [Ford, W.L. in Handbook of Experimental Immunology edit. by.: D.M. Weir. Blackwell Scientific Publications, Oxford, 1978. p. 23.6] (5 x 10³) were distributed into wells of round-bottom microtitre plates (Nunc) and incubated in the presence or absence of various dilutions of antigen, mitogen, lymphokine or other agent of interest. Cells were cultured in a atmosphere of 5% CO₂ in air for 72 hours after which ³H-Thymidine (0.5 uC1/well; 2.0 Ci/mM sp.act., New England Nuclear) was added 6 hours before termination of culture. The cells were harvested with a multiple automatic harvester, and ³H-thymidine incorporation assessed by liquid scintillation counting. ³H thymidine incorporation was determined relative to control values in the absence of inhibitor. Inhibitor was added to give a final concentration of 1 x 10⁻⁴M, but lower concentrations can be used.

### HIV gene replication

We examined the effect of H-Ala-boroPro on HIV-1 replication in vitro. The rational for these experiments comes from the reported connection between T-cell activation, IL-2 production, and HIV replication and expression of HIV proteins. For example, inductive signals associated with HIV replication include mitogens, antigens, lymphokines, and transcriptions factors such as NF-kB, all of which have been shown to be associated with induction of IL-2 production, T-cell activation, or both.

Cell lines used in the present studies include A3.5 cells (a monocyte cell line which is CD4+, HLA-DR+, and CD3-) and peripheral blood mononuclear cells (PBMC). The A3.5 cells grow continuously in culture without exogenous growth factors. PBMC cells require IL-2 for propagation in vitro. Cells were infected with HIV-1IIIB at a multiplicity of infection (moi) of 5 x 10⁻⁴ tissue culture infectious dose 50 (TCID50)/cell for both the A3.5 cells and the PMBC cells. Dilutions of inhibitor were made in RPMI-1640 and subsequently passed through a 0.22 um filter. At the start of each experiment, 1 x 10⁶ cells/well, in 24-2311 plates, were infected with HIV-1IIIB at the moi indicated above. Inhibitor was added simultaneously at the appropriate dilutions. All cultures were maintained at 5% CO₂ and 37°C in RPMI-1640 supplemented with penicillin, streptomycin, L-glutamine, hepes buffer, and 20% heat-inactivated fetal calf serum. Cell counts and viability were determined by trypan blue exclusion. Culture supernatants were harvested and assayed for HIV-1 p24 antigen by ELISA (NEN-DuPont, Boston, MA). Fresh media and inhibitor were added on each day. For PBMC cultures, cells were collected from HIV-1 seronegative donors and stimulated with PHA-P (Difco, Detroit, MI; 10 µg/ml) and 10% IL-2 (Electronnucleonics, Silver Spring, MD) 3 days prior to infection with HIV-1. PBMC cultures for all experiments included uninfected and infected cells without inhibitor, uninfected cells with inhibitor at the various concentrations, and infected cells in the presence of 1 um zidovudine (azidothvmidine, AZT).

With A3.5 cells H-Ala-boroPro suppresses HIV below detectable levels in a manner similar to the anti-HIV effect of AZT at 1 um. Similar results were observed with the PBMC cells. Thus, inhibitors of this invention have an anti HIV effect. Cell viability assays show that these inhibitors are not cytotoxic even at relatively high concentration (10-3 M for A3.5 cells).

### Determination of DP IV Activities in Biological Samples

The ability to determine DP IV activities associated with cells and tissues is highly desirable. For example, it will permit correlations to be made between level of inhibition of DP IV and the magnitude of the observed biological affect, e.g., on cell proliferation, and IL-2 production. Such correlation is helpful in establishing whether or not the biological affect is due to inhibition of DP IV. We have found that such determinations can be reproducibly and reliably made using the readily available chromogenic substrates for DP IV: X-Pro-p-nitroanilides and X-Pro-7-amino-4-trifluoromethyl coumarins (AFC). The AFC substrates are fluorescent and thus provide greater sensitivity. DP IV activity is measured as release of p-nitroanilide spectrophotometrically at 410nM, or using X-Pro-AFC derivatives and measuring fluorescence at 505nM. Reduction in activity in the presence of inhibitor provides an easy test for inhibitory activity.

### Use

The inhibitory compounds can be administered in an effective amount either alone or in combination with a pharmaceutically acceptable carrier or diluent.

The above inhibitory compounds are useful for treatment of a wide variety of disease; for example, an autoimmune disease, the pathogenesis of which is dependent on T cell activity. DP IV plays a role in such autoimmune disease and inhibition of DP IV activity allows regulation of the progress of the disease. Such diseases include arthritis, rejection of transplanted organs, as well as SLE and AIDS. When administered to mammals (e.g., orally, topically, intramuscularly, intraperitoneally, intravenously, parenterally, nasally or by suppository), the inhibitory compounds of this invention enhance the ability of, e.g., the immune system of the mammal, to fight the disease.

Inhibitors of DP IV can suppress IL-2 production and thus diseases in which the production of IL-2 is altered may be treated by use of these inhibitors. These inhibitors can also delay catabolism of growth hormone releasing factor, and block DPIV activity of amoebae and microbial pathogens to allow an immune system to act more efficiently.

The inhibitory compounds or compositions can be administered alone or in combination with one another, or in combination with other therapeutic agents. The dosage level may be between 1 - 500 mg/kg/day.

### Other Embodiments

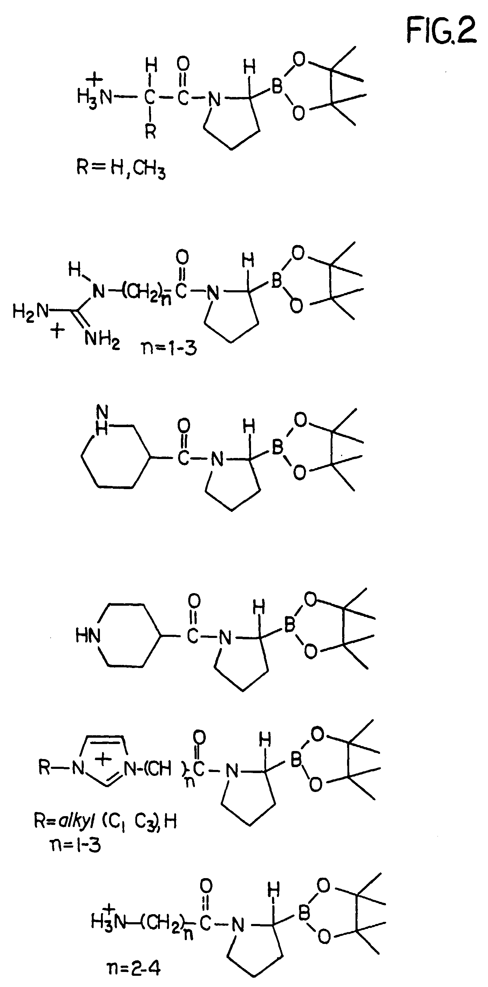

Other embodiments are within the following claims. For example, other inhibitors can be created which mimic the structure of Ala-boroPro. Examples of such inhibitors are shown in Fig. 2 and include Ala-boroPro. These inhibitors generally have a boroPro group, or its equivalent, described above in the Summary of the Invention, and a positively charged amine group. The inhibitors are designed so that minimal interaction of the amine and boroPro groups occurs, and thus no cyclic structure is formed at pH 7.0. These inhibitors interact and/or bind with DPIV, and thereby reduce the DPIV enzymatic activity toward a normal substrate. These inhibitors are synthesized by procedures well known to those of ordinary skill in this art.

## Claims

1. Use of a compound having the formula 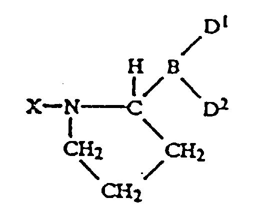 where each of D1 and D², independently, is a hydroxyl group or a group which is capable of being hydrolysed to a hydroxyl group in aqueous solution at physiological pH; and X comprises an amino acid or a peptide which mimics the site of a substrate recognised by a post prolyl cleaving enzyme for the preparation of a medicament for the treatment of transplant rejection or an autoimmune disease other than AIDS.

2. Use of a compound as defined in Claim 1 for the preparation of a medicament for the treatment of arthritis.

3. Use of a compound as defined in Claim 1 for the preparation of a medicament for the treatment of systemic lupus erythematosus.

## Patentansprüche

1. Verwendung einer Verbindung der Formel 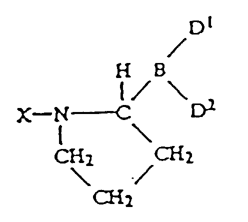 worin jeder der Substituenten D¹ und D² unabhängig eine Hydroxylgruppe oder eine Gruppe, die in wäßriger Lösung bei physiologischem pH zu einer Hydroxylgruppe hydrolisierbar ist, bedeutet und X eine Aminosäure oder ein Peptid umfaßt, welche bzw. welches die Stelle eines Substrats imitiert, die von einem post-Prolyl-Spaltungsenzym erkennbar ist, zur Herstellung eines Arzneimittels zur Behandlung von Transplantatabstoßung oder einer Autoimmunkrankheit, ausgenommen von AIDS.

2. Verwendung einer Verbindung nach Anspruch 1 zur Herstellung eines Arzneimittels zur Behandlung von Arthritis.

3. Verwendung einer Verbindung nach Anspruch 1 zur Herstellung eines Arzneimittels zur Behandlung von systemischem Lupus erythematosus.

## Revendications

1. Utilisation d'un composé ayant la formule : 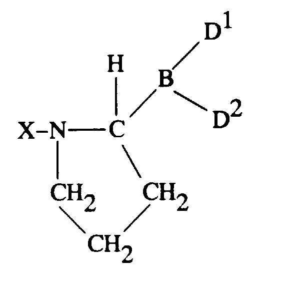 dans laquelle D¹ et D² représentent chacun indépendamment un groupement hydroxyle ou un groupement qui est capable d'être hydrolysé en un groupement hydroxyle dans une solution aqueuse au pH physiologique; et X comprend un acide aminé ou un peptide qui imite le site d'un substrat reconnu par une enzyme coupant après le prolyle pour la préparation d'un médicament destiné au traitement de rejet de greffon ou d'une maladie autoimmune autre que le SIDA.

2. Utilisation d'un composé selon la revendication 1 pour la préparation d'un médicament destiné au traitement de l'arthrite.

3. Utilisation d'un composé selon la revendication 1 pour la préparation d'un médicament destiné au traitement du lupus érythémateux aigu disséminé.
